# DEMANDE DE BREVET EUROPEEN

(11) **EP 3 763 297 A1**
(43) Date de publication de la demande: **13.01.2021**
(21) Numéro de dépôt: 19185399.3
(22) Date de dépôt: 10.07.2019
(51) Int. Cl.: A61B 17/04

(54) **DISPOSITIF D'ANCRAGE OSSEUX**

(71) Demandeur: Keri Medical SA, 1227 Les Acacias (CH)
(72) Inventeur: PRANDI, Bernard, 6006 Lucerne (CH); MOTTET, Julie, 74930 Pers-Jussy (FR); MOTTE, Damien, 74000 Annecy (FR)
(74) Mandataire: Micheli & Cie SA

(57) **Abrégé**

L'invention concerne un dispositif d'ancrage osseux (1) comprenant une ancre à visser (2) et au moins un fil de suture (3) monté sur l'ancre (2), dans lequel :
- l'ancre (2) comprend un corps (4) s'étendant selon un axe longitudinal (A) entre une extrémité proximale (4a) agencée pour coopérer avec un instrument de pose et une extrémité distale (4b), ledit corps (4) présentant un trou central (8) traversant selon ledit axe longitudinal (A), et une surface externe présentant un filetage (10) destiné au vissage de l'ancre (2) dans un os, l'ancre (2) comprenant également un support de fil de suture (3) autour duquel est enroulé le fil de suture (3) pour remonter dans le trou central (8) du corps (4) de l'ancre (2),
- le fil de suture (3) présente deux extrémités (3a) qui ressortent par l'extrémité proximale (4a) du corps (4) de l'ancre (2), au moins l'une des deux extrémités (3a) du fil de suture (3) comportant une aiguille (14).

Ledit support de fil de suture (3) est constitué par un pion (12) rapporté et rendu solidaire du corps (4) de l'ancre (2), monté dans le trou central (8) dudit corps (4) de l'ancre (2) du côté de son extrémité distale (4b), ledit pion (12) s'étendant selon l'axe longitudinal (A) et comportant une tête (16) agencée pour supporter le fil de suture (3) et un pied (18) agencé pour assurer le guidage du pion (12) dans le trou central (8), la tête (16) et le pied (18) du pion (12) étant agencés pour former des canaux (28) pour le passage du fil de suture (3) et comprenant des moyens de positionnement du pion (12) dans le trou central (8).

## Description

La présente invention concerne un dispositif d'ancrage destiné à être fixé dans un os lors d'une intervention relevant de la chirurgie orthopédique et notamment un dispositif tel qu'une ancre de suture.

Les lésions ligamentaires et tendineuses sont parmi les affections les plus fréquemment rencontrées en orthopédie et peuvent toucher un grand nombre d'articulations différentes comme celles de la main et du poignet. L'un des traitements connus de ces lésions consiste à venir refixer les tendons ou ligaments endommagés à leur place sur l'os grâce à une ancre et à un fil de suture. Pour ce faire, un perçage est réalisé dans l'os, l'ancre est ancrée dans l'os et un fil qui lui est accroché permet ensuite de suturer le tissu endommagé. Il existe des ancres impactées, vissées ou «à noeud» qui peuvent-être résorbables ou non résorbables. L'invention concerne en particulier une ancre de suture de type vissée non résorbable.

Lorsqu'une telle chirurgie concerne plus particulièrement la main et le poignet, une des difficultés est que la main possède des petits os, comme la dernière phalange des doigts. Cette phalange présente une forme irrégulière avec un faible diamètre (4 à 6 mm) non constant. Cela oblige à utiliser une ancre elle-même de petite taille et de prévoir un perçage diamétralement petit et peu profond. L'ancre utilisée doit donc être relativement compacte. Toutefois, tout en étant de petite taille, l'ancre doit malgré tout permettre le passage d'au moins un fil de suture, en particulier quand le fil de suture est terminé par une ou deux aiguilles. En général, un fil de suture avec aiguilles est préparé au préalable en sertissant des aiguilles aux extrémités du fil, l'ensemble étant ensuite conditionné en milieu propre puis stérilisé dans l'attente de son utilisation, et notamment de son montage avec une ancre en usine, l'ancre étant alors livrée avec un fil terminé par des aiguilles pré-monté au chirurgien.

Il existe par exemple des ancres à visser comprenant un corps fileté présentant à son extrémité proximale un chas de très petites dimensions et à travers lequel le fil de suture est enfilé. L'inconvénient de ce type d'ancre est que le fil de suture est très exposé et risque d'être abimé voire coupé par les bords du chas. De plus, le chas est trop petit pour permettre de passer un fil de suture sur lequel sont déjà serties des aiguilles, de sorte que cette ancre est réservée aux chirurgies par arthroscopie.

Certaines ancres à visser, comme celles décrites dans les brevets US 9,775,702 ou US 8,882,801, comprennent un corps cylindrique creux, fileté, présentant à son extrémité distale une découpe obtenue par moulage, formant un support autour duquel un fil de suture est enroulé puis passé à l'intérieur du corps cylindrique creux, les deux extrémité du fil de suture ressortant par l'extrémité proximale de l'ancre. Comme dans le dispositif précédent, l'inconvénient de ce type d'ancre est que le fil de suture est très exposé et risque d'être abimé voire coupé par les bords de la découpe. De plus, le passage formé par la découpe est trop petit pour permettre de passer un fil de suture sur lequel sont déjà serties des aiguilles, de sorte que ces ancres sont réservées aux chirurgies par arthroscopie.

Il existe également une ancre telle que proposée par exemple dans le brevet EP 1 584 296. Cette ancre à visser comprend une cavité au fond de laquelle est disposé un support s'étendant transversalement par rapport à l'axe longitudinal de l'ancre et maintenu par deux ouvertures prévues dans les faces latérales de l'ancre. Le fil de suture est passé autour du support, ses deux extrémités ressortant de l'ancre par son extrémité proximale. Toutefois, dans ce dispositif, les ouvertures pour le passage du support percées dans les faces latérales de l'ancre abiment le filetage de l'ancre à visser. De ce fait, la tenue de l'ancre dans l'os est moindre. De plus, ce dispositif ne permet pas de passer un fil de suture terminé par des aiguilles déjà serties en raison des dimensions intérieures de l'ancre et du positionnement du support dans la cavité de l'ancre. Il est donc réservé aux chirurgies par arthroscopie.

La présente invention vise à remédier à ces inconvénients en proposant un dispositif d'ancrage osseux pouvant être utilisé en chirurgie « à ciel ouvert », avec un fil de suture présentant à ses extrémités des aiguilles de suture pré-montées.

La présente invention vise également à proposer un dispositif d'ancrage osseux permettant un ancrage simple et de qualité, notamment pour des refixations tendineuses ou ligamentaires de la main et du poignet, bien que l'ancre soit de très petites dimensions adaptées aux petits os de la main et du poignet.

A cet effet, la présente invention concerne un dispositif d'ancrage osseux comprenant une ancre à visser et au moins un fil de suture monté sur l'ancre, dans lequel :
- l'ancre à visser comprend un corps s'étendant selon un axe longitudinal entre une extrémité proximale agencée pour coopérer avec un instrument de pose et une extrémité distale, ledit corps présentant un trou central traversant selon ledit axe longitudinal, et une surface externe présentant un filetage destiné au vissage de l'ancre dans un os, l'ancre à visser comprenant également un support de fil de suture autour duquel est enroulé le fil de suture pour remonter dans le trou central du corps de l'ancre
- le fil de suture présente deux extrémités qui ressortent par l'extrémité proximale du corps de l'ancre.

Selon l'invention, ledit support de fil de suture est constitué par un pion rapporté et rendu solidaire du corps de l'ancre, monté dans le trou central dudit corps de l'ancre du côté de son extrémité distale, ledit pion s'étendant selon l'axe longitudinal et comportant une tête agencée pour supporter le fil et un pied agencé pour assurer le guidage du pion dans le trou central, la tête et le pied du pion étant agencés pour former des canaux pour le passage du fil de suture et comprenant des moyens de positionnement du pion dans le trou central. De plus, au moins l'une des deux extrémités du fil de suture comporte une aiguille.

Un tel dispositif d'ancrage osseux permet de mettre à disposition d'un chirurgien souhaitant réaliser une chirurgie de la main « à ciel ouvert », une ancre de suture déjà munie d'un fil de suture comportant à ses extrémités des aiguilles de suture serties préalablement, tout en étant de très petite taille, adaptée à celle de la main.

La présente invention concerne également un équipement d'ancrage osseux comprenant un instrument de pose et un dispositif d'ancrage osseux tel que défini ci-dessus, pré-monté sur ledit instrument de pose, ledit instrument de pose comprenant un manche et une tête terminée à son extrémité distale par un embout monté dans l'extrémité proximale du corps de l'ancre, ledit embout présentant un orifice central dans lequel passe le fil de suture.

Selon l'invention, la tête de l'instrument de pose présente des ouvertures latérales agencées pour permettre un passage latéral du fil de suture de part et d'autre de la tête de l'instrument de pose en direction du manche, et le manche comprend des moyens de fixation des extrémités du fil de suture portant au moins une aiguille.

Un tel équipement d'ancrage osseux permet d'utiliser une ancre de suture déjà munie d'un fil de suture terminé par des aiguilles de suture, pré-montée en position sur son instrument de pose, facilitant ainsi le travail du chirurgien, en particulier lors d'une chirurgie de la main « à ciel ouvert ».

D'autres caractéristiques et avantages de la présente invention apparaîtront à la lecture de la description détaillée suivante d'un mode de réalisation de l'invention, donné à titre d'exemple non limitatif, et faite en référence aux dessins annexés dans lesquels :
- la figure 1 représente une vue en coupe longitudinale de l'ancre du dispositif d'ancrage osseux selon l'invention ;
- la figure 2 représente une vue de dessus du dispositif d'ancrage osseux selon l'invention ;
- la figure 3 représente une vue en perspective du corps de l'ancre du dispositif d'ancrage osseux selon l'invention ;
- la figure 4 représente une vue en coupe longitudinale du corps de l'ancre de la figure 3 ;
- la figure 5 représente une vue en perspective du pion de l'ancre du dispositif d'ancrage osseux selon l'invention ;
- la figure 6 représente une vue de face du corps et du pion avant assemblage pour former l'ancre du dispositif d'ancrage osseux selon l'invention ;
- la figure 7 est une vue en perspective de l'extrémité de l'instrument de pose ;
- la figure 8 est une vue en perspective de l'extrémité de l'instrument de pose portant le dispositif d'ancrage osseux selon l'invention ; et
- la figure 9 est une vue de dessus de l'instrument de pose portant le dispositif d'ancrage osseux selon l'invention.

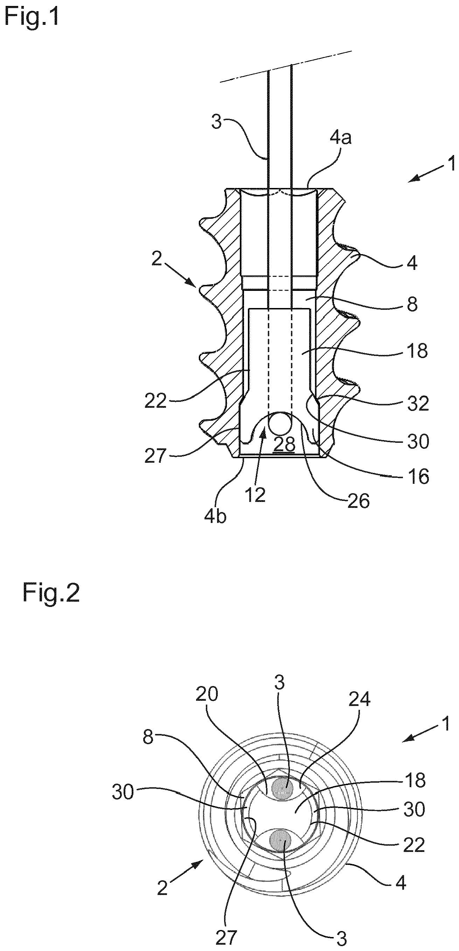

En référence aux figures 1 et 2, il est représenté un dispositif d'ancrage osseux 1 selon l'invention, ledit dispositif d'ancrage osseux comprenant une ancre à visser 2 et au moins un fil de suture 3 monté sur l'ancre 2.

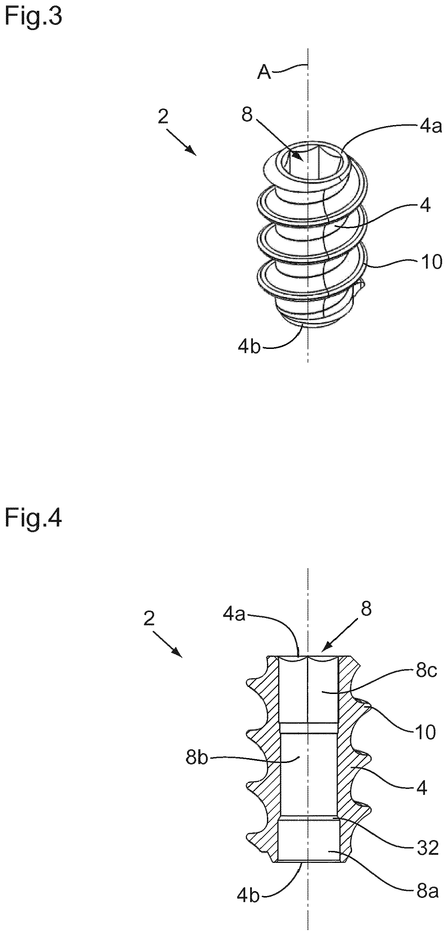

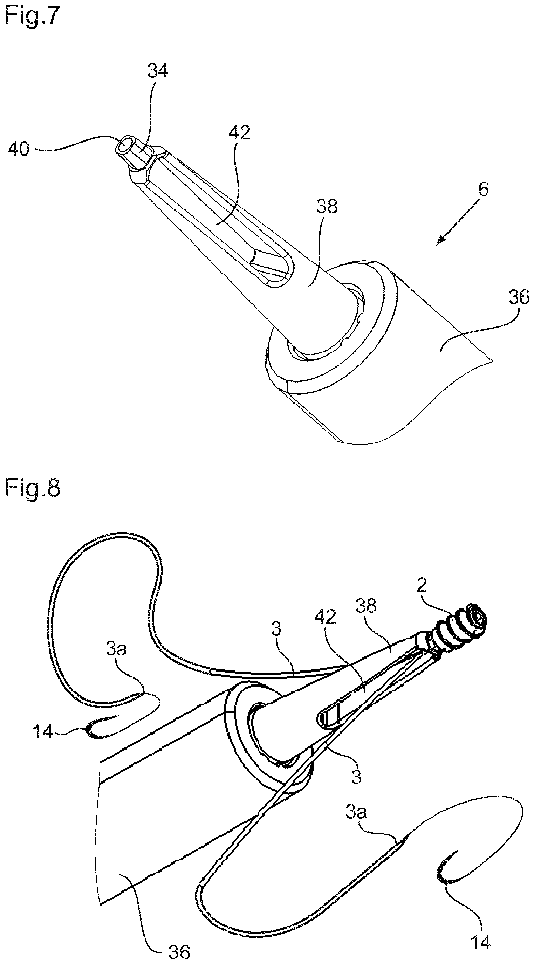

Plus spécifiquement en référence également aux figures 3 et 4, l'ancre à visser comprend un corps 4 cylindrique creux, s'étendant selon un axe longitudinal A, sa longueur étant supérieure à son diamètre extérieur. Par exemple son longueur est environ de 1.5 à 2 fois son diamètre extérieur. L'ancre 2 est une ancre de suture à visser notamment destinée à être utilisée pour refixer un tissu tel qu'un tendon ou un ligament sur un os, typiquement sur une phalange distale (P3) d'un doigt de la main, de sorte que le corps 4 de l'ancre 2 présente une très petite taille, avec par exemple une longueur de l'ordre de 4 mm. Le corps 4 s'étend entre une extrémité proximale 4a agencée pour coopérer avec un instrument de pose 6 qui sera décrit ultérieurement (cf. figures 7 et 8), et une extrémité distale 4b destinée à être insérée en premier dans l'os.

Le corps 4 étant creux, il présente un trou central 8 traversant selon l'axe longitudinal A, et s'étendant entre l'extrémité proximale 4a et l'extrémité distale 4b du corps 4.

Le corps 4 présente également, sur sa surface externe, un filetage hélicoïdal 10 destiné au vissage de l'ancre 2 dans un os. De préférence, le filetage 10 s'étend sur toute la longueur du corps 4, de son extrémité proximale 4a à son extrémité distale 4b.

L'ancre 2 comprend également un support 12 de fil de suture autour duquel le fil de suture 3 est enroulé pour former une boucle du côté de l'extrémité distale 4b du corps 4 pour remonter dans le trou central 8 dudit corps 4, les deux extrémités 3a du fil de suture 3 ressortant par l'extrémité proximale 4a dudit corps 4 de l'ancre 2. Le dispositif d'ancrage osseux 1 selon l'invention étant destiné à une chirurgie « à ciel ouvert », au moins l'une des deux extrémités 3a du fil de suture 3 comporte une aiguille de suture 14 (cf. figure 8). De préférence, chacune des deux extrémités 3a du fil de suture comporte une aiguille de suture 14.

Conformément à l'invention, le support de fil de suture 12 est constitué par un pion rapporté et rendu solidaire du corps 4 de l'ancre 2, monté dans le trou central 8 dudit corps 4 de l'ancre 2 du côté de son extrémité distale 4b. Ledit support ou pion 12 s'étend selon l'axe longitudinal A, sa dimension longitudinale étant supérieure à sa dimension transversale. Par exemple, sa longueur est environ 1.2 fois sa dimension transversale maximum.

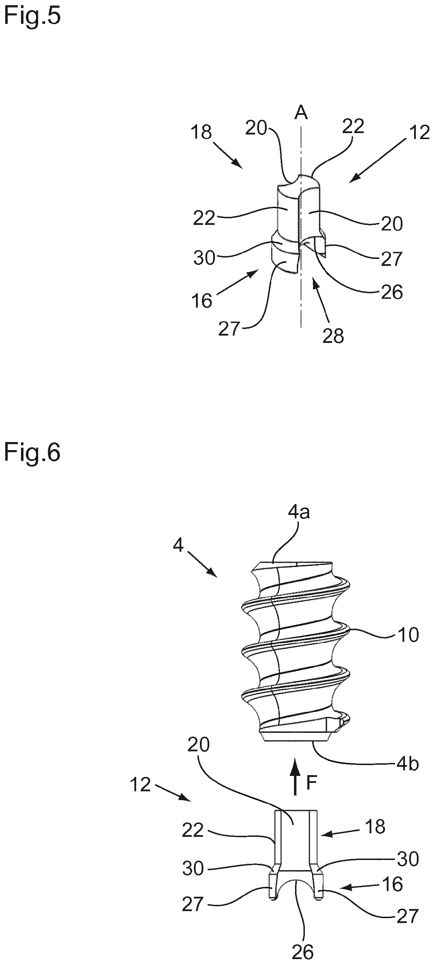

Plus spécifiquement en référence aux figures 5 et 6, le pion 12 comporte une tête 16 agencée pour supporter le fil de suture 3 et un pied 18 agencé pour assurer le guidage du pion 12 dans le trou central 8 du corps 4 de l'ancre 2, la tête 16 et le pied 18 du pion 12 étant agencés pour former des canaux pour le passage du fil de suture 3 lorsqu'il remonte dans le trou central 8.

A cet effet, le pied 18 du pion 12 se présente sous la forme d'un cylindre allongé s'étendant selon l'axe longitudinal A et à la surface duquel ont été pratiquées deux creusures latérales 20 longitudinales et symétriques par rapport à l'axe A de manière à former deux faces avant et arrière opposées concaves et deux parois latérales opposées 22 convexes. Les creusures latérales 20 sont agencées pour former avec la paroi du trou central 8 des canaux 24 pour le passage du fil de suture 3 remontant dans ledit trou central 8, comme le montre la figure 2. D'une manière avantageuse, les creusures 20 présentent des surfaces arrondies pour faciliter le passage du fil de suture 3 et ne pas l'abimer.

La tête 16 se présente sous la forme d'un cylindre dans lequel a été creusée une gorge 26 pour former, à l'extrémité distale 4b du corps 4 de l'ancre 2, de l'avant vers l'arrière, un canal ouvert traversant 28 pour le passage du fil de suture 3, et délimité de part et d'autre par deux parois latérales 27 convexes, de forme extérieure cylindrique. La gorge 26 s'étend perpendiculairement aux creusures 20 prévues sur le pied 18 de sorte que le canal ouvert 28 communique avec les canaux 24 formés par le pied 18 et la paroi du trou central 8. D'une manière avantageuse, la gorge 26 de la tête 16 présente une surfaces arrondie pour faciliter le passage du fil de suture 3 et ne pas l'abimer.

De plus, la tête 16 et le pied 18 du pion 12 comprennent des moyens de positionnement du pion 12 dans le trou central 8, agencés notamment pour contrôler le positionnement longitudinal du pion 12 dans le corps 4 de l'ancre 2.

A cet effet, la tête 16 du pion 12 présente une section transversale de dimensions (c'est-à-dire de diamètre) supérieures à celles de la section transversale du pied 18 du pion 12 de manière à former entre la tête 16 et le pied 18 du pion 12, et plus précisément entre une paroi latérale 27 de la tête 16 et la paroi latérale 22 du pied 16 contiguë, au moins un premier épaulement périphérique 30 formant les moyens de positionnement du pion 12 dans le trou central 8. De préférence, le pion 12 comprend deux épaulements 30 symétriques par rapport à l'axe A formant des moyens de positionnement autobloquants du pion 12 dans le corps 4 de l'ancre 2, comme cela sera décrit ci-dessous.

En parallèle, comme le montre la figure 4, le trou central 8 comprend un premier alésage 8a débouchant à l'extrémité distale 4b et agencé pour recevoir la tête 16 du pion 12, ledit premier alésage 8a présentant une première section transversale. Le trou central 8 comprend également un deuxième alésage 8b communiquant avec le premier alésage 8a, agencé pour recevoir le pied 18 du pion 12, ledit deuxième alésage 8b présentant une deuxième section transversale de dimensions inférieures à celles de la première section transversale du premier alésage 8a de manière à former entre les premier et deuxième alésages 8a, 8b un deuxième épaulement périphérique 32 agencé pour coopérer avec le premier épaulement périphérique 30 du pion 12 en formant une butée de positionnement.

D'une manière avantageuse, le trou central 8 du corps 4 de l'ancre 2 comprend un troisième alésage 8c communiquant avec le deuxième alésage 8b et débouchant à l'extrémité proximale 4a du corps 4, ledit troisième alésage 8c étant agencé pour recevoir l'instrument de pose 6, et présentant une troisième section transversale différente de la deuxième section transversale du deuxième alésage 8b. De préférence, la troisième section du troisième alésage 8c est de forme complémentaire à celle d'un embout mâle 34 prévu sur l'instrument de pose 6, formant une empreinte rétentive permettant d'appliquer un couple via un instrument de pose, comme cela sera décrit ci-après. Par exemple la troisième section peut être de forme hexagonale ou toute autre forme polygonale appropriée ou de forme Torx.

Avant leur assemblage pour former l'ancre 2, le corps 4 et le pion 12 sont positionnés de sorte que le pion 12 se situe en face du trou central 8 comme le montre la figure 6. Le fil de suture 3 est plié sensiblement en deux pour former au centre une boucle et est introduit par l'extrémité proximale 4a dans le trou central 8 du corps 4 de l'ancre 2. Le fil de suture 3 terminé à ses extrémités 3a par les aiguilles 14 est assez long pour que, une fois plié en deux et introduit dans le trou central 8, les extrémités 3a portant les aiguilles 14 restent suffisamment à distance du corps 4. La boucle formée par le fil de suture 3 est passée dans le canal ouvert 28 pour s'appuyer dans le fond de la gorge 26 de la tête 16 du pion 12, les deux brins du fil de suture 3 passant de part et d'autre du pied 18 sur les creusures 20. Puis le pion 12, avec le fil de suture 3, est monté, selon la flèche F, dans le trou central 8 jusqu'à ce que les premiers épaulements 30 du pion 12 butent contre le deuxième épaulement 32 du trou central 8. La tête 16 est logée dans le premier alésage 8a et le pied 18 est logé dans le deuxième alésage 8b. Les deux brins du fil de suture 3 remontent dans le trou central 8 en direction de l'extrémité proximale 4a du corps 4 par les canaux 24, puis traversent le troisième alésage 8c, les extrémités 3a du fil de suture 3 qui portent les aiguilles 14 restant à l'extérieur du corps 4 de l'ancre 2, comme le montre la figure 1. Le pion 12 est ensuite assemblé au corps 4, dans sa partie distale, par exemple par chassage, au moyen d'une presse.

La première section transversale du premier alésage 8a du trou central 8 est dimensionnée pour que les parois latérales convexes 27 de la tête 16 épousent un secteur circulaire de forme complémentaire de la paroi du premier alésage 8a venant en contact.

La première section transversale du premier alésage 8a du trou central 8 est dimensionnée pour représenter environ la moitié du diamètre extérieur du corps 4.

D'une manière avantageuse, les moyens de positionnement, c'est-à-dire les premiers épaulements 30 du pion 12 sont agencés et disposés par rapport au deuxième épaulement 32 de manière à positionner le pion 12 entièrement dans le trou central 8 du corps 4 de l'ancre 2, le canal ouvert 28 formé par la tête 16 pour le passage du fil de suture 3 étant entièrement dans le trou central 8. Ainsi le fil de suture 3 est entièrement protégé à l'intérieur du corps 4 et ne risque pas d'être abimé lors du vissage de l'ancre 2.

De préférence, la dimension longitudinale du pion 12 représente au moins 1/3 de la dimension longitudinale du trou central 8 du corps 4 de l'ancre 2. Plus particulièrement, la longueur du pied 18 est égale environ à 1 à 2 fois la longueur de la tête 16 de manière à assurer le guidage et le bon positionnement du pion 12 lorsqu'il est monté dans le trou central 8. Les dimensions des pièces sont très petites de sorte qu'un tel guidage au moyen du pied 18 est nécessaire pour garantir une mise en place correcte selon l'axe longitudinal A du pion 12 dans le corps 4 de l'ancre 2.

Le corps de l'ancre et le pion sont réalisés dans tout matériau compatible avec une chirurgie. Par exemple, ils peuvent être réalisés tous les deux en alliage de titane biocompatible.

La présente invention concerne également un équipement d'ancrage osseux comprenant un instrument de pose 6 et un dispositif d'ancrage osseux 1 tel que décrit ci-dessus, pré-monté sur ledit instrument de pose 6.

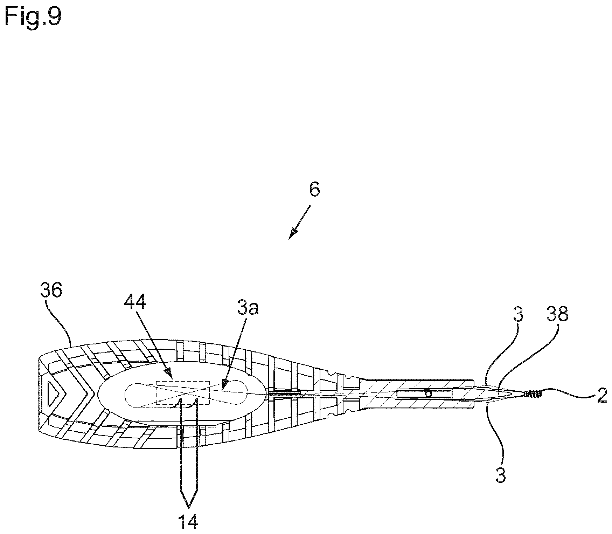

En référence aux figures 7 à 9, ledit instrument de pose 6 comprend un manche 36 portant une tête 38 terminée à son extrémité distale par un embout 34 agencé pour être monté dans l'extrémité proximale 4a du corps 4 de l'ancre 2, et plus particulièrement dans le troisième alésage 8c de forme complémentaire du trou central 8, comme décrit ci-dessus. Sur la figure 7, l'embout 34 présente une section transversale de forme octogonale, qui correspond à une autre variante de réalisation, différente de la forme hexagonale requise pour coopérer avec le troisième alésage 8c de section transversale hexagonale représenté sur les figures 1 à 6. Il est bien évident que toute autre forme d'embout 34 peut être utilisée, le troisième alésage 8c ayant une forme complémentaire de manière à constituer une empreinte rétentive.

L'embout 34 présente un orifice central 40 dans lequel passe les deux brins du fil de suture 3 sortant du trou central 8 par l'extrémité proximale 4a du corps 4 de l'ancre 2.

Selon l'invention, la tête 38 de l'instrument de pose 6 présente au moins deux ouvertures latérales 42 agencées pour permettre un passage latéral des brins du fil de suture 3 de part et d'autre de la tête 38 de l'instrument de pose 6 en direction du manche 36.

De plus, le manche 36 comprend des moyens de fixation 44 des extrémités 3a du fil de suture 3 portant au moins une aiguille 14. Ces moyens de fixation 44 sont par exemple une carte autour de laquelle les extrémités 3a portant les aiguilles 14 sont enroulées.

D'une manière avantageuse, les ouvertures latérales 42 de la tête 38 de l'instrument de pose 6 sont longitudinales, en s'étendant selon l'axe longitudinal de l'instrument de pose 6, qui correspond à l'axe longitudinal A du corps 4 de l'ancre 2. Les ouvertures latérales 42 se font face et sont de préférence disposées de manière symétrique par rapport audit axe longitudinal du côté de l'extrémité distale de la tête 38 de l'instrument de pose 6, à proximité immédiate de l'embout 34.

De préférence, les dimensions de chaque ouverture latérale 42, et en particulier la dimension longitudinale, sont choisies de manière à permettre à chaque aiguille 14 de passer aisément par ladite ouverture latérale 42 lorsque l'ancre 2 est monté sur l'embout 34 ainsi que lorsque l'instrument de pose 6 est retiré de l'ancre 2.

De même, la dimension longitudinale de l'embout 34 est choisie très inférieure à la longueur de l'aiguille 14 afin de permettre à ladite aiguille 14 de passer aisément de l'embout 34 à l'ouverture latérale 42 lorsque l'ancre 2 est monté sur l'embout 34 et de l'ouverture latérale 42 à l'embout 34 lorsque l'instrument de pose 6 est retiré de l'ancre 2.

Le dispositif d'ancrage osseux 1 selon l'invention peut être utilisé en chirurgie « à ciel ouvert », avec un fil de suture présentant à ses extrémités des aiguilles de suture pré-montées.

La mise en place du dispositif d'ancrage selon l'invention est standard avec la réalisation d'un pré-trou suivi du vissage de l'ancre, et ne nécessite pas de description détaillée.

A l'extrémité distale de l'ancre, le fil de suture est protégé à l'intérieur du corps de l'ancre, de sorte qu'il n'est pas abimé. De plus, le corps de l'ancre est intègre, et ne présente aucun orifice susceptible d'abimer son filetage. Ainsi, le vissage de l'ancre dans l'os n'est pas affaibli.

Le dispositif d'ancrage osseux 1 selon l'invention permet un ancrage simple et de qualité, et est particulièrement approprié pour des refixations tendineuses ou ligamentaires de la main et du poignet, bien que l'ancre soit de très petites dimensions adaptées aux petits os de la main et du poignet.

De plus, l'équipement d'ancrage osseux selon l'invention permet de pré-monter l'ancre sur l'instrument de pose et de retirer ledit instrument de pose après la mise en place de l'ancre dans l'os, tout en préservant l'intégrité du fil de suture et de ses aiguilles de suture.

## Revendications

1. Dispositif d'ancrage osseux (1) comprenant une ancre à visser (2) et au moins un fil de suture (3) monté sur l'ancre (2), dans lequel :
- l'ancre (2) comprend un corps (4) s'étendant selon un axe longitudinal (A) entre une extrémité proximale (4a) agencée pour coopérer avec un instrument de pose (6) et une extrémité distale (4b), ledit corps (4) présentant un trou central (8) traversant selon ledit axe longitudinal (A), et une surface externe présentant un filetage (10) destiné au vissage de l'ancre (2) dans un os, l'ancre (2) comprenant également un support de fil de suture (3) autour duquel est enroulé le fil de suture (3) pour remonter dans le trou central (8) du corps (4) de l'ancre (2),
- le fil de suture (3) présente deux extrémités (3a) qui ressortent par l'extrémité proximale (4a) du corps (4) de l'ancre (2),
**caractérisé en ce que** ledit support de fil de suture (3) est constitué par un pion (12) rapporté et rendu solidaire du corps (4) de l'ancre (2), monté dans le trou central (8) dudit corps (4) de l'ancre (2) du côté de son extrémité distale (4b), ledit pion (12) s'étendant selon l'axe longitudinal (A) et comportant une tête (16) agencée pour supporter le fil de suture (3) et un pied (18) agencé pour assurer le guidage du pion (12) dans le trou central (8), la tête (16) et le pied (18) du pion (12) étant agencés pour former des canaux (24, 28) pour le passage du fil de suture (3) et comprenant des moyens de positionnement du pion (12) dans le trou central (8),
et **en ce qu'**au moins l'une des deux extrémités (3a) du fil de suture (3) comporte une aiguille (14).

2. Dispositif d'ancrage osseux (1) selon la revendication 1, **caractérisé en ce que** le pied (18) du pion présente des creusures (20) latérales longitudinales agencées pour former avec la paroi du trou central (8) des canaux (24) pour le passage du fil de suture (3) remontant dans ledit trou central (8).

3. Dispositif d'ancrage osseux (1) selon l'une des revendications précédentes, **caractérisé en ce que** la tête (16) présente une gorge (26) formant un canal ouvert (28) pour le passage du fil de suture (3) communiquant avec les canaux (24) formés par le pied (18).

4. Dispositif d'ancrage osseux selon les revendications 2 et 3, **caractérisé en ce que** les creusures (20) du pied (18) et la gorge (26) de la tête (16) présentent des surfaces arrondies.

5. Dispositif d'ancrage osseux (1) selon l'une des revendications précédentes, **caractérisé en ce que** la tête (16) du pion (12) présente une section transversale de dimensions supérieures à celles de la section transversale du pied (18) du pion (12) de manière à former entre la tête (16) et le pied (18) du pion (12) au moins un premier épaulement périphérique (30) formant les moyens de positionnement du pion (12) dans le trou central (8).

6. Dispositif d'ancrage osseux (1) selon la revendication 5, **caractérisé en ce que** le trou central (8) du corps (4) de l'ancre (2) comprend un premier alésage (8a) débouchant à l'extrémité distale (4b) et agencé pour recevoir la tête (16) du pion (12), ledit premier alésage (8a) présentant une première section transversale, et un deuxième alésage (8b) communiquant avec le premier alésage (8a), agencé pour recevoir le pied (18) du pion (12), ledit deuxième alésage (8b) présentant une deuxième section transversale de dimensions inférieures à celles de la première section transversale du premier alésage (8a) de manière à former entre les premier (8a) et deuxième (8b) alésages un deuxième épaulement périphérique (32) agencé pour coopérer avec le premier épaulement périphérique (30) du pion (12) en formant une butée de positionnement.

7. Dispositif d'ancrage osseux (1) selon l'une des revendications 5 et 6, **caractérisé en ce que** le trou central (8) du corps (4) de l'ancre (2) comprend un troisième alésage (8c) communiquant avec le deuxième alésage (8b) et débouchant à l'extrémité proximale (4a), agencé pour recevoir l'instrument de pose (6), ledit troisième alésage (8c) présentant une troisième section transversale différente de la deuxième section transversale du deuxième alésage (8b).

8. Dispositif d'ancrage osseux (1) selon la revendication 7, **caractérisé en ce que** la troisième section du troisième alésage (8c) est de forme complémentaire à celle d'un embout (34) prévu sur l'instrument de pose (6).

9. Dispositif d'ancrage osseux (1) selon l'une des revendications précédentes, **caractérisé en ce que** les moyens de positionnement du pion (12) sont agencés pour positionner le pion (12) entièrement dans le trou central (8) du corps (4) de l'ancre (2), le canal ouvert (28) formé par la tête (16) pour le passage du fil de suture (3) étant entièrement dans le trou central (8).

10. Dispositif d'ancrage osseux (1) selon l'une des revendications précédentes, **caractérisé en ce que** le pion (12) présente une dimension longitudinale supérieure à sa dimension transversale, et **en ce que** la dimension longitudinale du pion (12) représente au moins 1/3 de la dimension longitudinale du trou central (8) du corps (4) de l'ancre (2).

11. Dispositif d'ancrage osseux (1) selon l'une des revendications précédentes, **caractérisé en ce que** le filetage (10) sur la surface externe du corps (4) de l'ancre (2) s'étend de l'extrémité proximale (4a) à l'extrémité distale (4b) du corps (4).

12. Equipement d'ancrage osseux comprenant un instrument de pose (6) et un dispositif d'ancrage osseux (1) selon l'une des revendications précédentes pré-monté sur ledit instrument de pose (6), ledit instrument de pose (6) comprenant un manche (36) et une tête (38) terminée à son extrémité distale par un embout (34) monté dans l'extrémité proximale (4a) du corps (4) de l'ancre (2), ledit embout (34) présentant un orifice central (40) dans lequel passe le fil de suture (3), **caractérisé en ce que** la tête (38) de l'instrument de pose (6) présente des ouvertures latérales (42) agencées pour permettre un passage latéral du fil de suture (3) de part et d'autre de la tête (38) de l'instrument de pose (6) en direction du manche (36), et **en ce que** le manche (36) comprend des moyens de fixation (44) des extrémités (3a) du fil de suture (3) portant au moins une aiguille (14).

13. Equipement d'ancrage osseux selon la revendication 12, **caractérisé en ce que** lesdites ouvertures latérales (42) de la tête (38) de l'instrument de pose (6) sont longitudinales et disposées du côté de l'extrémité distale de la tête (38) de l'instrument de pose (6), à proximité immédiate de l'embout (34).

14. Equipement d'ancrage osseux selon l'une des revendications 12 et 13, **caractérisé en ce que** la dimension longitudinale de l'ouverture latérale (42) est choisie de manière à permettre à l'aiguille (14) de passer par ladite ouverture latérale (42).

15. Equipement d'ancrage osseux selon l'une des revendications 12 à 14, **caractérisé en ce que** la dimension longitudinale de l'embout (34) est choisie très inférieure à la longueur de l'aiguille (14) afin de permettre à ladite aiguille (14) de passer de l'embout (34) à l'ouverture latérale (42) et de l'ouverture latérale (42) à l'embout (34).
